Europäisches Patentamt

European Patent Office

Office européen des brevets

(19)

(11) Publication number: **0 401 949**
**A2**

## (12) EUROPEAN PATENT APPLICATION

(21) Application number: **90300124.6**

(22) Date of filing: **05.01.90**

(51) Int. Cl.⁵: **A61F 13/02**

(30) Priority: **05.06.89 US 361414**

(43) Date of publication of application:
**12.12.90 Bulletin 90/50**

(84) Designated Contracting States:
**AT BE CH DE DK ES FR GB GR IT LI LU NL SE**

(71) Applicant: **BERTEK INC.**
**Jonergin Drive**
**Swanton Vermont 05488(US)**

(72) Inventor: **Wick, John J.**
**7 Country Crossing**
**Williston Vermont 05492(US)**

(74) Representative: **Beresford, Keith Denis Lewis**
**et al**
**BERESFORD & Co. 2-5 Warwick Court High**
**Holborn**
**London WC1R 5DJ(GB)**

(54) **Thin film applicator.**

(57) Applicators for applying film layers to the skin of a patient are disclosed which include a liner layer with a releasable surface, a film layer releasably affixed to the releasable surface of the liner layer, and a carrier layer in contact with the other side of the film layer and including at least two portions of a carrier layer (32a, 32b) covering respective portions of the film layer, with each of these carrier layer portions including tab portions (32d, 32b) free of adhesive overlapping each other (34), so that after the liner layer has been removed and the film layer has been applied to the patient, the carrier layer can be readily removed by pulling on these tab portions. An active agent carrier may be combined with said applicators.

EP 0 401 949 A2

## THIN FILM APPLICATOR

The present invention relates to the application of film layers to the skin or mucosa of a patient. More particularly, the present invention relates to devices for the handling of film layers to be applied to the skin or mucosa of a patient. Still more particularly, the present invention relates to the simultaneous application of film layers and devices for the administration of an active agent to the host.

The application of bandages and other types of dressings to a patient's skin for healing purposes has been used for many years. In the distant past, it was believed that optimum healing would occur under the natural protective cover of dried wound exudate, namely a scab. Thus, dressing materials were typically composed of gauze or fabric used over both traumatic and surgical wounds for protection from gross particulate contamination as well as to promote drying and scab formation.

Not until the early 1970's was it determined that, contrary to more established thought, wounds actually heal significantly faster and with less scar formation under a film which maintains a well-balanced, moist environment, such as that which naturally occurs under a blister. This alteration of the perception of wound healing processes led to the introduction, beginning in 1981, of a new category of "moist environment" synthetic transparent adhesive dressings which were based on adhesive coated film layers extruded or cast from high-moisture-vapor-permeable, elastomeric resins. Initially, polyurethanes were used. In the several years since their initial commercial acceptance, these products have achieved wide-spread acceptance in the medical community based on both clinical performance and cost effectiveness.

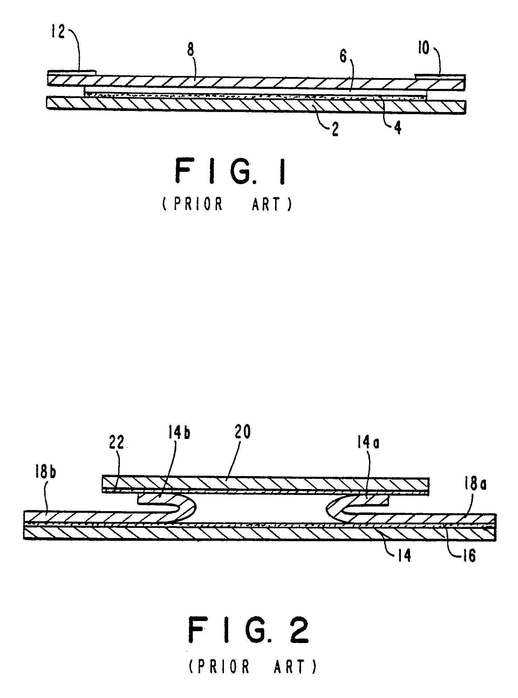

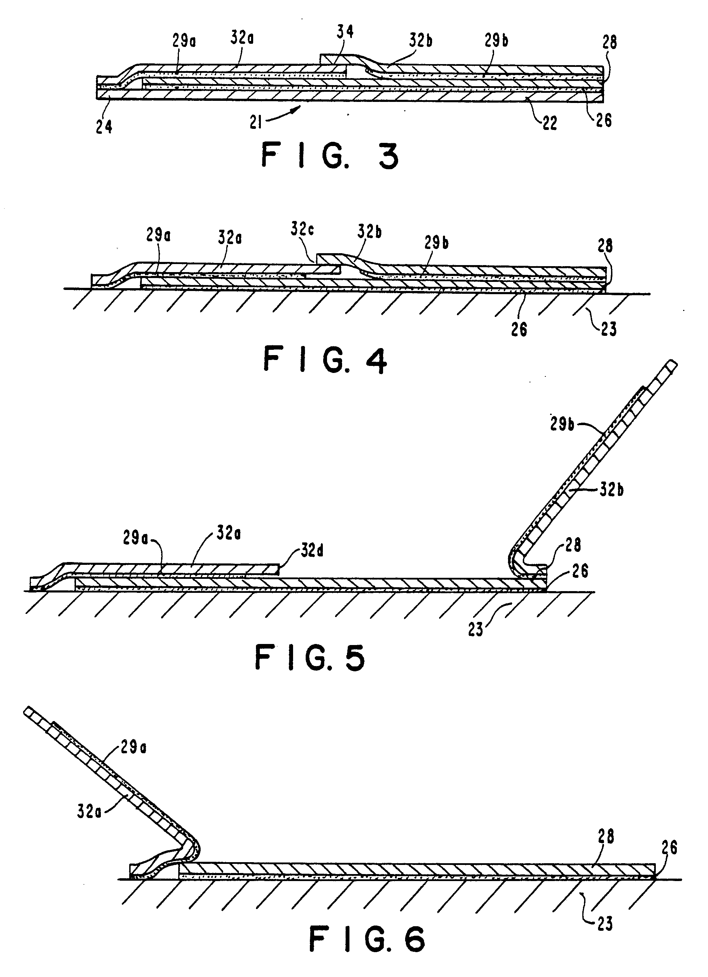

An example of an adhesive medical or surgical dressing which includes a moisture-vapor-permeable-pressure-sensitive material is disclosed in Hodgson, U.S. Patent Nos. Re. 31886 and 31887. The adhesive medical or surgical dressing is specifically shown in FIG. 5 of these patents, and includes a dressing pad 18 on the adhesive side thereof. There are also a number of other such wound dressing products currently on the market. These include products having a configuration such as that shown in FIG. 1 hereof. These include, for example, wound dressings being sold by Ferris Corporation of Burr Ridge, Illinois under the mark DYNADERM™, which include a liner layer 2, a film layer 6 adhesively and releasably secured to the liner layer 2 by releasable adhesive layer 4, and a backing layer 8. The backing layer 8 includes color coded tabs 10 and 12 at either end thereof. This device is thus employed by first pulling on tab 10 and peeling backwards so as to separate the backing layer 8 and film layer 6, along with adhesive layer 4, from the liner layer 2. The film and adhesive layers can then be applied to the skin, and it is then necessary to pull on the second tab 12 to remove the backing layer 8 from the film layer 6, leaving the film layer on the skin. A similar such wound dressing is also sold by The Kendall Company of Boston, Massachusetts, under the mark POLYSKIN™.

Other commercial wound dressing products include a product sold by Johnson & Johnson Products Inc. of New Brunswick, New Jersey under the mark BIOCLUSIVE, and have construction such as that shown in FIG. 2 hereof. In this case, the product includes a film layer 14, which includes an adhesive layer 16 on its inner surface. On that inner surface there are a pair of folded backing members 18a and 18b, and a central backing member 20 with an inner adhesive layer 22 thereon. To use this device, it is thus necessary to initially remove the central backing member 20, to then apply the central portion of the film layer 14 with the adhesive coating 16 to the skin of the patient, and separately remove the pair of outer backing members 18a and 18b by pulling on the folded-over portions 19a and 19b thereof, respectively.

In each of these cases, however, application of a smooth film layer at a proper location is quite difficult to attain. Firstly, in connection with the types of products represented by FIG. 1, particularly upon application of the film layer 6 with its adhesive layer 4 to the skin, removal of the backing layer 8 becomes quite difficult, and in fact when tab 12 is lifted, this generally results in wrinkling, removal or displacement of the corresponding end of film layer 2 adjacent thereto. In addition, it is difficult to apply to the film layer 2 to awkward body locations, such as behind the shoulder, and at the same time remove the tab 12 and apply the film layer 2 smoothly and evenly. On the other hand, as for the type of product shown in FIG. 2, a significant difficulty with the utilization of this device is that after the central portion layer 14 has been applied to the skin it is obvious that two hands are required to remove both of the separate backing members 18a and 18b and to attempt to smoothly apply the film layer 14 on each side thereof. Once again, smooth application of the film layer becomes extremely difficult, if not impossible.

There have also been considerable commercial developments in connection with transdermal administration systems. These include Ciba-Geigy's TRANSDERM®-NITRO and TRANSDERM®-V systems which have been approved for transdermal administration of nitroglycerine and scopolamine, respectively. These prior devices for the controlled, continuous metering of drugs and the like include a reservoir for the drug as well as a permeable adhesive layer for maintaining the transdermal patch or the like on the skin or mucosa of a patient. A significant improvement in this field is represented by U.S. Patent No. 4,573,996, which is seepage-resistant during use and thus eliminates prior problems of contamination which were previously encountered with such devices.

A continuing problem which has been generated by these devices related to their overall handling and more particularly to the need to maintain the transdermal patches in contact with the skin for extended time periods. In such situations, even where highly effective adhesives have been utilized, a tendency remained for the adhesive to become less effective with time and use, and for the patch to eventually fall off.

The above problems, including the difficulty in smoothly and cleanly applying film layers, and the difficulties in handling and maintaining transdermal patches on the skin for long periods of time, have been considerably lessened by the invention disclosed in copending U.S. Patent Application Serial No. 07'239,592, filed on September 1, 1988, and assigned to the assignee of the present application. However, efforts have continued to further improve on these problems, and to more rapidly facilitate the smooth and easy application of film layers to the patient's skin, and preferably in a manner which can be done with one hand, and at the same time achieve essentially perfect film application in each case.

According to the present invention, there is provided an applicator for applying a film layer to the skin or mucosa of a patient comprising a liner layer including a first surface and a second surface, said first surface of said liner layer comprising a releasable surface, a film layer including a first surface and a second surface, said first surface of said film layer being releasably disposed on said releasable surface of said liner layer, and a carrier layer disposed in contact with said second surface of said film layer, said carrier layer comprising a first portion and a second portion, said first portion of said carrier layer covering a first portion of said second surface of said film layer, and including a first adhesive portion and a first tab portion free of adhesive, said second portion of said carrier layer covering a second portion of said second surface of said film layer, and including a second adhesive portion and a second tab portion free of adhesive, said first and second tab portions overlapping each other, whereby upon removal of said liner layer and application of said first surface of said film layer to said skin or mucosa of said patient said carrier layer can be readily removed from said second surface of said film layer by removal of said first and second portions of said carrier layer by pulling on said first and second tab portions, respectively.

In order that the invention may be fully understood, it will now be described with reference to the accompanying drawings in which:

FIG. 1 is a side, elevational, sectional view of an applicator device of the prior art;

FIG. 2 is a side, elevational, sectional view of another applicator device of the prior art;

FIG. 3 is a side, elevational, sectional view of one embodiment of an applicator of the present invention;

FIG. 4 is a side, elevational, sectional view of the applicator of FIG. 3 with the liner layer removed, applied to the skin.

FIG. 5 is a side, elevational, sectional view of the applicator of FIG. 4, with a portion of the first portion of the carrier layer being removed therefrom;

FIG. 6 is a side, elevational, sectional view of the applicator of FIG. 5, with the second portion of the carrier layer being removed therefrom;

FIG. 7 is a side, elevational, sectional view of another embodiment of the applicator of the present invention for applying a film layer and an active agent carrier layer therewith;

FIG. 8 is a top, elevational view of an applicator device of the present invention, with the liner layer removal initiated;

FIG. 9 is a top, elevational view of the applicator device of FIG. 9 with the first portion of the carrier layer being removed after application to the patient; and

FIG. 10 is a top, elevational view of the film layer of the applicator device of FIG. 9 applied to the patient.

The present invention can be more fully appreciated with reference to the above-noted figures and to the following detailed description thereof. In connection with the foregoing figures, like reference numerals will refer to like portions thereof.

Referring first to the applicator of the present invention shown in FIG. 3, this embodiment is intended to apply a film layer 28 to the skin or mucosa of a host or patient. Thus, the applicator 21 of this invention includes a film layer 28 sandwiched therein which itself comprises an extremely thin and flimsy layer of material which is preferably breathable and non-occlusive in nature. The film layer 28 is composed of a material which would be extremely difficult, if not impossible, to handle by itself, i.e., apart from the applicator of the present invention. The film layer 28, which is shown after having been applied to the skin 23 of the patient in FIG. 6, in this embodiment is intended to act as a wound dressing. This film layer 28, or "skin," is thus extremely flimsy material which preferably allows air and moisture vapor to pass therethrough, but which will not permit the passage of bacteria or other undesired elements or materials. It is preferred that film layer 28 thus be composed of various thin, plastic materials, or it can comprise an extremely thin foil layer, or layer of other non-woven materials, and most particularly it will comprise a material which is sufficiently thin and flexible to be conformable to the skin. This material will thus preferably be thinner than about 2 mils, preferably less than about 1.5 mils, and most preferably 1 mil or less.

These film layers 28 are preferably thermoplastic materials which are at least partially elastomeric in nature. They will therefore exhibit a high degree of elongation (preferably greater than about 130% elongation), and will thus exhibit excellent conformability characteristics without having the tendency to exhibit significant memory characteristics, although they will have some degree of recovery when stretched, for example. In terms of being breathable films it is preferred that these materials, in addition to permitting air to pass therethrough, will also permit moisture vapor to pass through them, at least more readily than is the case with materials such as polyethylene, for example. All of these film layers must be occlusive, at least with respect to particulates, in order to protect the wound, etc. However, their overall occlusive characteristics can then vary, depending upon the particular environment in which it is intended that they will ultimately be used. In general, however, it is preferred that films be employed which are permeable to various glycols, such as polyethylene glycols, but rather more occlusive films can also be employed in selected circumstances, including, for example, 1 and 2 mil layers of ethylenevinyl acetate copolymers, or various nylon or polyester films. In addition, laminated or coated films can also be utilized, such as by employing one of the non-occlusive films discussed above which is fully or partially coated with an occlusive film.

The various thermoplastic films themselves can generally be produced with either a matte, glossy, or a clear surface, which is obtained by selection or modification of the surface of the chilling roller generally used downstream of the film extruder from which the film is extruded. These films can also include various colors, such as skin color, as well as fillers, such as $TiO_2$, clay, or other such materials for the purpose of rendering the film opaque, and various organic additives, odor inhibitors, and/or various medications, etc. which can be applied directly onto the surface thereof.

From the commercial viewpoint, one of the most successful high moisture vapor permeable medical grade elastomeric films has been one of a series of products marketed by Dupont under the designation "Medifilm 800." These films are extruded from a class of elastomeric resins which are polyether block amides, commercially designated by the trademark PEBAX. The structure of these polymers can be generally represented by the formula:

$$HO \{C-PA-C-PE-O\}NH$$
$$\overset{\|}{O} \quad \overset{\|}{O} \quad \overset{\|}{C}$$

in which PA represents a relatively rigid polyamide segment and PE represents a relatively soft polyether segment. In this manner the extruded film products have high strengths in terms of high tear and abrasion resistance and at the same time provide a high degree of comfort or conformability, as well as moisture vapor permeability. The physical properties of two typical medical grade PEBAX films having a thickness of 1 mil are set forth in TABLE 1 herein.

TABLE 1

| | FILMS | |
|---|---|---|
| PROPERTIES | MEDIFILM 810 | MEDIFILM 827 |
| Tensile strength | | |
| - psi (ASTM D882) | 3120 | 2200 |
| % Elongation | 430 | 800 |
| Modulus @ 50% elongation | 1600 | 900 |
| Initial tear resistance | | |
| - lbs. (ASTM D-1004) | 0.65 | 0.6 |
| MUTR -g/m2/24 hrs. | | |
| (ASTM E-96) 37.8 C/90% R.H. | 1675 | 2200 |

In addition, other such film layers 28 can comprise thermoplastic polyurethanes which also meet the above requirements. These include such commercial polyurethane compositions as Dow Chemical Company's PELLETHANE, including its 2363-80AE grade thereof; K.J. Quinn's Q-THANE; B. F. Goodrich's ESTANE; Mobay Chemical Company's TXIN; and others. Furthermore, these film layers 28 can also comprise various polyesters, such as the copolymers of various cyclic polyesters including Dupont's HYTREL, including its 4056 grade thereof, and General Electric's LOMOD, both of which are copolymers of polyether prepolymers and polybutylene terephthalate and polyisobutyl terephthalate, respectively, as well as Eastman Chemical's PCCE.

In order to adequately maintain the film layer 28 on the surface of the skin of the host, the only additional element required is a layer of adhesive 26 on the underside of film layer 28 as can best be seen in FIGS. 5 and 6.

As for adhesive layer 26 itself, it should have properties and characteristics which maintain the film layer 28 on the skin surface as, for example, with the devices in the prior art. It is primarily only necessary therefor that the coefficient of adhesion as between film layer 28 and the skin surface 23 to which it is being applied be sufficient to overcome the adhesive or other force which affixes the film layer 28 to the carrier layer 32 which is provided for the purpose of carrying film layer 28 prior to its application. The carrier layer 32 in the case of the embodiment of FIG. 3 is, in accordance with an essential element of the present invention, comprised of two carrier layer portions, 32a and 32b. In connection with the application of film layer 28 above, an acrylic copolymer adhesive, such as Avery Chemical Company's AS-351 HSX, can be utilized as the adhesive layer 26, preferably at a coating weight of between about 25 and 35 g/m². This pressure sensitive adhesive is a cross-linkable polymer which dries to provide a permanently tacky film having a total solids content of about 52%, a Brookfield viscosity (LVT/04/12 RPM @ 25° C) of from about 15,000 to 25,000 cps and a weight per gallon of about 7.4 lbs. It can also be diluted with hexane or toluene to a desired solids and/or viscosity range, particularly for use in conventional coating equipment. Again, other such adhesives which are particularly useful together with drug delivery systems are discussed more fully below.

Referring again to FIG. 3, the applicator 21 shown therein includes a pair of carrier layer portions 32a and 32b, each of which includes a corresponding adhesive portion 29a and 29b, respectively, by which they are affixed to a portion of the outer surface of the film layer 28. However, it is also possible to affix these portions of the carrier layer to the surface of film layer 28 by other means of temporarily adhering these layers, such as by heat sealing or other such means for temporary lamination thereof.

A most important consideration in the selection and design of these portions of the carrier layer 32a and 32b is to be sure that the film layer 28 is adhered to these portions of the carrier layer 32a and 32b with sufficient strength to maintain it thereon when the film layer 28 is exposed or uncovered by initial removal of the liner layer 22 (see discussion below), while at the same time this bond must be sufficiently weak in relative terms so as to readily peel off or be removable from the film layer 28 once that layer has been applied to the skin 23 by means of the adhesive layer 26 discussed above.

Of most significance, however, is the configuration of these portions of the carrier layer 32a and 32b. In the embodiment shown in FIG. 3 each thus covers essentially one-half of the surface of film layer 28, with the exceptions discussed below. In their central region these portions of the carrier layer 32a and 32b thus overlap, at the central portion 34 shown in FIG. 3. However, the portion of overlap between the portions of carrier layer 32a and 32b, namely as at 34 in FIG. 3, do not include adhesive means in the embodiment shown in FIG. 3. As will be discussed below, it is possible to include some adhesive within this portion 34 for purposes of creating a seal, but it is nevertheless necessary to maintain the end portions 32d and 32c, respectively, of the portions 32a and 32b of the carrier layer free of adhesive or the like for purposes to be discussed in more detail below. Beyond that, however, the portions of adhesive layers 29a and 29b as shown in FIG. 3 can be a pressure-sensitive adhesive, such as a cross-linkable acrylic copolymer, etc., or a heat-sealable adhesive. Where the adhesive is an acrylic copolymer it can, for example, be essentially the same acrylic copolymer as that which is the adhesive layer 26 for applying the film layer 28 to the skin surface, including the materials such as the Avery Chemical Company AS-351 HSX adhesive, discussed above. Other such adhesives which can also be used for these purposes include an acrylic pressure-sensitive adhesive sold by National Adhesives under the designation DURO-TAK 80-1054. This adhesive has a solids content of 47.5%, a viscosity of 3,000 cps, and a plasticity (Williams) of 2.9 mm. It is generally used with a solvent system including ethyl acetate, heptane, isopropyl alcohol and toluene. Another such adhesive is sold by Monsanto under the designation GELVA Multipolymer Emulsion 2484, and comprises a stable aqueous acrylic emulsion pressure-sensitive adhesive having a solids content of 59%, and a viscosity of 1,500-Z, 300 cps. With these various adhesives the adhesion of this layer can be reduced as compared to adhesive layer 26 by the addition of a greater amount of cross-linking additives thereto and/or by utilizing different coating weights and/or viscosities therefor. When the portions of the carrier layer 32a and 32b are paper, for example, it is also possible to adhere the film layer 28 thereto by means of coating its surface with a heat-sealable adhesive layer 29a and 29b and then heat sealing these two layers together. Alternatively, film layer 28 and the portions of carrier layer 32a and 32b can be laminated together, such as when they are both plastic layers, or where they are different layers such as plastic film layer 28 and paper portions of carrier layer 32a and 32b, by flowing the plastic film layer 28 onto the surface of the paper carrier layer portions 32a and 32b--i.e., as an extrusion laminate.

The applicator 21 of the present invention also includes a liner layer 22 which is also initially but temporarily (releasably) affixed to the underside of the film layer 28 which includes adhesive layer 26.

This liner layer 22 is intended to protect the film layer 28 prior to its use, and to render it transportable while not interfering with its ultimate application, and at the same time protecting its adhesive layer 26. At the same time the liner layer 22 must also be easily removable or releasable from the film layer 28 and its adhesive layer 26. Doing so thus exposes the underside of film layer 28 with adhesive layer 26 thereon for subsequent application to the skin of the host. It is thus preferable that the liner layer 22 include a release layer to permit its easy removal from the laminate composite of this device. The liner layer 22 can thus be composed of some of the same materials which are also suitable for use as the portions of the carrier layer 32a and 32b discussed above. This material, however, is made removable or releasable from the adhesive layers by, for example, treatment with silicone or other suitable coatings. It can therefore comprise various layers, including paper or paper-containing layers or laminates; various thermoplastics, such as extruded polyolefins, such as polyethylene; foil liners; or various laminates of these different layers.

As an example, a preferred liner layer 22 will thus comprise a silicone or teflon release-coated paper, but it can also comprise various polyester films; foil layers, preferably coated with a polymer such as polyethylene; other such layers, including fabric layers, coated or laminated to various polymers, as well as extruded polyethylene, polyethylene terephthalate, various polyamides, and the like. The selection of a particular liner layer 22 will also depend upon other ultimate requirements of the particular device in question, including whether there is a desire for a transparent or opaque liner, etc.

It can therefore be seen that although the adhesive layer 26 is present on at least a portion of the inner surface of the liner layer 22 for maintaining the various layers in contact in the configuration shown in FIG. 3, the entire liner layer 22 is "peelable," or releasable, by merely pulling apart the edge of liner layer 22 as shown at 24 in FIG. 3. At the same time, when this is done, the entire film layer 28 remains in position in contact with, and maintaining its original relationship with, the portions of the carrier layer 32a and 32b. Because of the above-discussed relationship between the adhesive materials in the adhesive layers 26 and 29a and 29b, respectively, they thus maintain that contact upon removal of liner layer 22.

To further facilitate initiation of separation of the liner layer 22 from the underside of the applicator 21, it is preferable that a tab portion 24 of the liner layer 22 be free of adhesive, i.e., free of adhesive layer 26. This tab portion can thus be used to initiate the effective separation of the liner layer 22. There are other methods, however, which can be used for the same purpose, such as extending the liner layer 22 beyond the end of the portion of carrier layer 32a so as to facilitate the grasping of same, or on the other hand, a slit or the like can be provided in one of these two layers, permitting it to be readily peeled back from the leading edge thereof. Other such methods will be apparent to those of ordinary skill in this art.

The two portions of the carrier layer 32a and 32b should be composed of a material which is also

EP 0 401 949 A2

flexible, but which has considerably more substance and strength than does film layer 28. It should, however, be flexible enough to generally follow the contour of the area of the host where the film layer 28 is to be applied. On the other hand, it should have enough strength and substance so as to serve its function of carrying film layer 28 without wrinkling, etc. The actual material from which the portions of carrier layer 32a and 32b can be produced can therefore include a variety of different materials. Some suitable materials for this layer include, for example, polyethylene, polypropylene, polyvinylidene chloride, polyethylene terephthalate, polyesters, polyamides, and others, as well as laminates of two or more of these layers with each other or with additional layers, such as foil, paper, various fabrics, etc., but in these cases preferably with the polymer layer on the inside, i.e.--in contact with and thereby carrying the film layer 28.

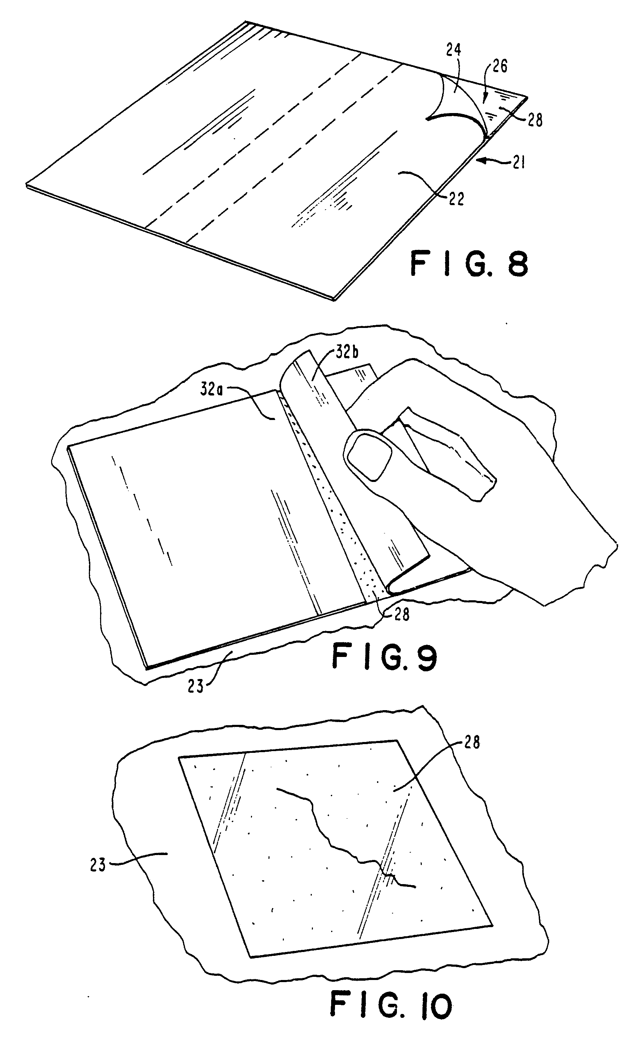

Reference is next made to the sequence showing application of the film layer 28 of the present invention to the skin 23 of a patient in FIGS. 3-6 hereof, as well as in FIGS. 8-10. The applicator 21 of the present invention is, as discussed in detail above, shown in FIG. 3. To initiate application of film layer 28 it is thus necessary to initially remove liner layer 22 to expose adhesive layer 26. As is also discussed above, this is facilitated by grasping tab portion 24 of the liner layer 22 and peeling it back, as can also be seen in FIG. 8. Because of the above-described nature of liner layer 22, which is releasable, it thus will be easily removed from the film layer 28 exposing adhesive layer 26, without effecting any change in the relationship between film layer 28 and the two portions of the carrier layer 32a and 32b. With the liner layer 22 fully removed and discarded, the remaining portion of the applicator 21 can then be applied to the skin by merely placing the adhesive layer 26 and its associated film layer 28 against the patient's skin 23 at the location desired, i.e., where the wound is to be covered, etc. This is easily accomplished, again by merely placing the respective areas of the adhesive layer 26 against the skin 23 and gently applying pressure against the outer surface of the portions of carrier layer 32a and 32b. This configuration can be seen in FIG. 4, positioned upon the patient's skin 23 as shown therein.

It is then necessary to remove the portions of carrier layer 32a and 32b, and their associated adhesive layers 29a and 29b, so as to leave the film layer 28 alone adhered to the skin 23. This is a simple step in accordance with the present invention, but at the same time it permits one to apply and maintain the film layer 28 in a wrinkle-free condition, without any unnecessary movement or removal of any portion of film layer 28 during this further procedure. In addition, because of the unique construction of the applicator of the present invention, this can be established on or at awkward body locations by using one hand alone, and again without causing any undue movement of film layer 28. Referring to FIGS. 5 and 9, one merely grasps the free end of the upper overlapping portion 32b of the carrier layer, and peels back into the configuration shown in FIG. 5. Since the tab portions at overlapping portion 34 of the two portions of carrier layer 32a and 32b are essentially adhesive-free (with the exception discussed below) and the outward end 32c of portion 32b is free of adhesive, it can easily be grasped and peeled backwards. Furthermore, since in this configuration the coefficient of adhesion of the adhesive layer 26 between the film layer 28 and the skin 23 is greater than the coefficient of adhesion of adhesive portion 29b as between the film layer 28 and the portion of carrier layer 32b, this action readily results in removal of this portion of carrier layer 32b. Furthermore, since this action is essentially from a central point on the film layer 28 to an outer point, there is no tendency to pull up or remove an end portion of the film layer 28. This can be contrasted, for example, to the prior commercial device shown in FIG. 1 where removal of the carrier layer 8 from its end portions results in awkward pulling of the film layer itself, wrinkling of same, and displacement thereof. This is a major advantage of the present invention.

The next step in removing the final portion 32a of the carrier layer can be see in FIG. 6. Thus, the inner end 32d of the portion of the carrier layer 32a, which is also free of adhesive, can thus be easily peeled back, preferably by the patient's same hand, into the configuration shown in FIG. 6. Once again, as in the case with FIG. 5, the placement of this end of this portion of carrier layer 32a on a central portion of the film layer 28 and peeling back towards the outer end of film layer 28 prevents untoward movement or removal of the film layer 28. This portion of the carrier layer 32a is thus now easily peeled back and removed, leaving the film layer 28 adhered to the desired location of the patient's skin 23 by means of adhesive layer 26 as can be seen in FIG. 10. No matter how thin and flimsy film layer 28 may be, that will not alter this procedure and/or prevent the applicator of the present invention from being smoothly, evenly and accurately placed in the desired location.

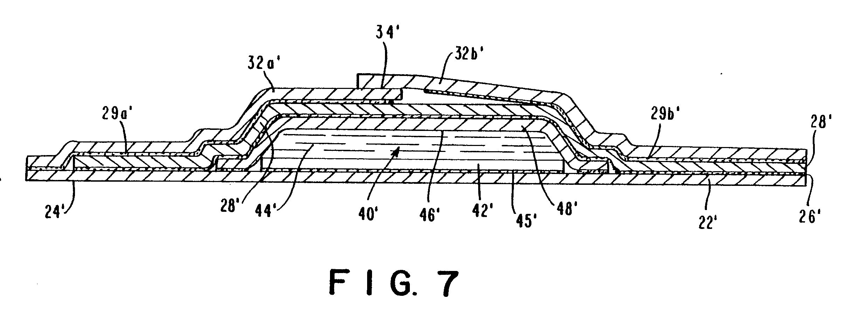

The present invention can also be utilized to transfer an active agent carrier to the skin or mucosa of a patient or host along with film layer 28. In this manner, the film layer 28 can in effect operate as a carrier for the active agent carrier itself, so that both are simultaneously applied to the skin in accordance with the same general procedure discussed above in connection with FIGS. 3-6. As can be seen in FIG. 7, the active agent carrier 40' is affixed to the underside of film layer 28 so that ultimately both film layer 28 and active agent carrier 40' will be applied to the skin of the host.

Referring specifically to FIG. 7, the active agent itself is maintained in a separate and distinct reservoir 44' created by a backing member 46' and a membrane 42' sealed to each other about the circumference or perimeter of the membrane, preferably by means of a heat seal. An active agent permeable adhesive layer 45' can be applied to the surface of the membrane 42' opposite the reservoir 44'. That is, reference can be made to the above discussion with respect to the adhesive layer 26 in the embodiment of FIGS. 3-6, which is interposed between the liner layer 22 and the bottom face of the film layer 28. In this case, the portion of that adhesive layer which is at the location of membrane 42' must be an active agent permeable adhesive layer, i.e., so that the active agent can permeate through that layer when it is eventually interposed between the active agent and the skin of the host into which it is intended to penetrate. However, the active agent permeable adhesive layer 45' should be limited to the area of the membrane 42', and the remaining portion of the adhesive layer 26' necessary for coating the inner surface of the liner layer 28' can comprise an active agent impermeable adhesive layer, which will be discussed in more detail below. The reason for this is that use of an active agent permeable adhesive over the entire surface of the liner layer 22', or at least beyond the periphery of the active agent carrier 40', would concomitantly increase the drug delivery surface and the treatment dosage for the particular drug delivery system in question would be undesirably altered thereby. Furthermore, it is preferred that the active agent impermeable adhesive layer around the periphery of the active agent carrier 44' in contact with the film layer 28' can be a strong adhesive in order to maintain the outer periphery of the film layer 28' in contact with the skin of the host. Indeed, in that case it is possible to eliminate the active agent permeable adhesive layer 45' completely, and merely utilize the surrounding active agent impermeable adhesive layer 26' to maintain the entire structure in place, i.e., the film layer 28' will maintain the active agent carrier 40' in place. It is still preferable, however, to employ a thin layer of active agent permeable adhesive layer 45' in contact with the active agent carrier, at least for insuring maintenance of contact between the active agent carrier and the skin of the host. For that purpose a contact-type adhesive, of essentially less strength, can be utilized.

As can be seen in the embodiment shown in FIG. 7, the active agent impermeable adhesive layer 26' which surrounds the active agent carrier 40' covers the entire inner surface of the film layer 28', i.e., thus affixing the inner surface of the film layer 28' to the outer surface of the backing member 48'. Thus, upon removal of the liner layer 22', the two adhesive layers can be applied to the skin. Furthermore, the film layer 28' and the impermeable adhesive layer 26' will form a pocket surrounding the backing member 48', the reservoir 44', the membrane 42', and the active agent permeable adhesive layer 45' so that when the device is adhered to the skin the active agent can be released through the membrane 42' and through the active agent permeable adhesive layer 45' to provide a continuous dose of the active agent therethrough, but cannot permeate through the film layer 28' or radiate outwardly through the active agent impermeable adhesive layer 26'. It is also possible to eliminate the backing member 48' so that the reservoir 44' is in this case created in the space between the active agent impermeable adhesive layer 26' and the membrane 42'.

The outer surface member, that is the member of the active agent carrier which is in contact with the film layer 28', be it a separate overlay covering layer or the backing member 48' described above, is preferably a thin film or sheet. In many instances, because of the area of skin to which the device is to be attached, the device is flesh colored for cosmetic reasons. Preferably, it is a clear polyester layer, which is occlusive with respect to the active agent or drug, but it can also be dyed various colors, or include printed matter thereon. The outer surface layer normally provides support and a protective covering for the device.

The outer surface layer is preferably made of a material or combination of materials which is substantially impermeable to the layer or layers with which it can be in contact, i.e., to the active agent, the impermeable and permeable adhesives, etc. However, a primary purpose is to prevent seepage of the active agent through the outer surface layer of the device so, if the outer surface layer is coated on the surface in contact with the remainder of the device with the active agent impermeable adhesive layer, this impermeable adhesive layer will perform this purpose even if the outer surface layer is not totally impermeable to the active agent. Thus, it is not necessary in all instances that the outer surface layer be impermeable to the active agent, although in most instances it normally is. By substantially impermeable we mean that the other components in contact with the layer or component under consideration will not appreciably permeate through such layer or component for the normal period of use and storage of the device, see the discussion of impermeability, *infra*.

The actual material used for the outer surface layer, i.e., the overlay covering layer and/or the backing member 48', will depend on the properties of the materials in contact therewith. Some suitable materials for the outer surface layer include, for example, cellophane, cellulose acetate, ethyl cellulose, plasticized vinyl acetate-vinyl chloride copolymers, ethylene-vinyl acetate copolymer, polyethylene terephthalate, nylon, polyethylene, polypropylene, polyvinylidene chloride, paper, cloth, and aluminum foil.

The material which forms the outer surface layer, either the overlay covering layer or the backing member, may be flexible or non-flexible. Preferably, a flexible outer surface layer is employed to conform to the shape of the body member to which the device is attached.

Preferably, the material which forms the overall covering layer and/or the backing member 48' is a film or a composite of films. The composite can be a metalized (e.g., aluminized) film or a laminate of two or more films or a combination thereof. For example, a laminate of polyethylene terephthalate and polyethylene or a polyethylene/metalized polyethylene terephthalate/polyethylene laminate can be employed. The preferred polymers include polyethylene, polypropylene, polyvinyl chloride and polyethylene terephthalate.

As mentioned above, a primary purpose of the active agent impermeable adhesive layer, such as layer 26', is to provide adhesion to the skin or mucosa, to prevent seepage of the active agent from the device during storage and use, and to maintain the overall film layer against the skin or mucosa surrounding the active agent carrier. Thus, any adhesive which performs these functions will be suitable for use in the present invention. The degree of impermeability of the active agent impermeable adhesive layer to the active agent will vary depending upon the active agent, carrier, transportation agent, etc. Preferably, the active agent impermeable adhesive layer is a pressure sensitive adhesive suitable for contact with the skin or mucosa, e.g., dermatologically acceptable. Examples of suitable pressure sensitive adhesive for use in the present invention as the active agent impermeable adhesive layer include natural rubber adhesives such as R-1072 from B. F. Goodrich Co., No. 735 from C. L. Hathaway, and No. 5702 from Evans-St. Clair; acrylic adhesives such as PS-41 from C. L. Hathaway, Vr-0833 from H. B. Fuller, Adcote 73A207A from Morton Chemical, Nos. 80-2404, 80-1054, 72-9056, and 72-9399 from National Starch, Nos. E-2015, E-2067 and E-1960 from Rohm & Haas, M-6112 from Uniroyal Inc. and Daratak 74 L from W. R. Grace; and synthetic rubber adhesives such as Jowatherm 270-00 and Jowatherm S-3202 from Jowat Corp. and 70-9416 from National Starch.

The width and thickness of the impermeable adhesive layer for contact with the skin or mucosa is that width and thickness which provides at least sufficient impermeability to the active agent (and if necessary to the other components of the device with which the impermeable adhesive layer is in contact) so that the active agent does not seep out of the device as explained above. Some suitable widths include 1/16 to 2 inches, and preferably 1/8 to 1 inches. In most instances, the width will be 1/4 to 1/2 inch depending on the specific use. The width and thickness need not be uniform and may vary around the perimeter of the device, e.g., to provide a specific geometric shape or to provide a tab for removal of a protective liner.

The active agent permeable adhesive layer, which is used primarily to maintain contact of the active agent carrier 40 with the skin, can thus have less adhesive strength. Therefore, the adhesive is preferably dermatologically acceptable, and it must, of course, be compatible with the active agent itself. It may also have to meet other requirements, such as resistance to alcohol or other solvents used in the particular active agent system in question. The active agent permeable adhesive layer is also preferably a pressure-sensitive adhesive. Any of the well-known, dermatologically acceptable, pressure-sensitive adhesives which permit drug migration therethrough can be used in the present invention. Some suitable permeable adhesives include acrylic or methacrylic resins such as polymers of esters of acrylic or methacrylic acid (e.g., n-butanol, n-pentanol, isopentanol, 2-methyl butanol, 1-methyl butanol, 1-methyl pentanol, 2-methyl pentanol, 3-methyl pentanol, 2-ethyl butanol, isooctanol, n-decanol, or n-dodecanol esters thereof), alone or copolymerized with ethylenically unsaturated monomers such as acrylic acid, methacrylic acid, acrylamide, methacrylamides, N-alkoxymethyl acrylamides, N-alkoxymethyl methacrylamides, N-t-butylacrylamide, itaconic acid, vinyl acetate, N-branched alkyl maleamic acids wherein the alkyl group has 10 to 24 carbon atoms, glycol diacrylates, or mixture of these monomers; natural or synthetic rubbers such as silicon rubber, styrene-butadiene rubber, butyl-ether rubber, neoprene rubber, nitrile rubber, polyisobutylene, polybutadiene, and polyisoprene; polyurethane elastomers; vinyl polymers, such as polyvinyl alcohol, polyvinyl ethers, polyvinyl pyrrolidone, and polyvinyl acetate; urea formaldehyde resins; phenol formaldehyde resins; resorcinol formaldehyde resins; cellulose derivatives such as ethyl cellulose, methyl cellulose, nitrocellulose, cellulose acetate butyrate and carboxymethyl cellulose; and natural gums such as guar, acacia, pectina, starch, destria, gelatin, casein, etc. The adhesives may also be compounded with tackifers and stabilizers as is well-known in the art.

The permeable adhesive layer preferably contains some of the active agent when the device is placed on the skin. This provides an initial drug presence at the skin or mucosa and eliminates delay in absorption of the active agent or in topical application, if that is desired. Thus, the drug is immediately available to the host. The initial drug presence may be due to the permeations through the membrane and/or to an amount of the drug mixed in with active agent permeable adhesive layer during manufacture, while on the other hand a surrounding layer of active agent impermeable adhesive serves to limit and clearly define the area of drug administration.

The amount of the active agent present in the permeable adhesive layer depends on the initial drug presence desired, e.g., for a pulse dosage. For example, U.S. Patent No. 4,031,894 discloses that 10-200 micrograms scopolamine base per $cm^2$ effective surface area is a suitable initial amount of active agent in the permeable adhesive layer.

The width and thickness of the permeable adhesive layer for contact with the skin or mucosa is that width and thickness which provides sufficient permeability to the active agent and a suitable surface area to allow the dosage rate desired to the skin or mucosa. These widths and thicknesses are conventional in the art and therefore need not be discussed in detail here.

The reservoir 44' is separated from the permeable adhesive layer by a membrane 42'. The membrane may be microporous in which case the pores become filled with active agent from the reservoir. The membrane may also function in any other way as long as the active agent permeates through the membrane at a suitable rate. The membrane and the permeable adhesive layer can be monolithic. In this instance, the surface of the membrane is treated to make it adhesive in nature so that it will adhere to the skin or mucosa but still provide membrane permeability characteristics.

The suitability of the rate of permeation of the active agent through the membrane depends on the desired dosage rate and the permeability of the active agent through the skin or mucosa, if transdermal type administration is desired. An effective amount of the active agent is contained in the reservoir to provide the desired dosage. Sometimes, the skin or mucosa itself determines the rate at which the active agent will be administered therethrough. In these latter instances, if the dosage rate through the skin or mucosa is the dosage rate desired, the membrane need not provide any limiting rate of permeation function but need only supply sufficient active agent to the skin or mucosa to allow the desired permeation through the skin or mucosa which itself determines the dosage rate at which the active agent will be absorbed by the host.

The materials suitable for use as the membrane are conventional in the art and need not be discussed in detail here. Some preferred materials for a separate membrane layer may be, for example, polypropylene, polycarbonates, polyvinyl chloride, cellulose acetate, cellulose nitrate, and polyacrylonitrile. Some suitable encapsulating membrane materials include, for example, hydrophobic polymers such as polyvinyl chloride either unplasticized or plasticized with long-chain fatty amides or other plasticizers, plasticized nylon, unplasticized soft nylon, silicone rubber, polyethylene, and polyethylene terephthalate. Hydrophilic polymers can also be employed such as esters of acrylic and methacrylic acid (e.g., as described in U.S. Patent Nos. 2,976,576 and 3,220,960 and Belgium Pat. No. 701,813); modified collagen; cross-linked hydrophilic polyether gels (e.g., as described in U.S. Patent No. 3,419,006); cross-linked polyvinyl alcohol; cross-linked partially hydrolized polyvinyl acetate; cellulosics such as methyl cellulose, ethyl cellulose, and hydroxyethyl cellulose; and gums such as acacia, carboxymethylcellulose, and carageenan alone or combined with gelatin.

The active agents suitable for use in this embodiment of the present invention may be, for example, systemic or topical drugs. Individual active agents or mixtures thereof, if desired, can be employed. Any drug which passes through the skin or mucosa can be employed for internal administration in the device of the invention, so long as the drug will pass through the permeable adhesive layer and the material forming the membrane or microcapsules.

Suitable systemic drugs for administration by the claimed device include those useful in treating emesis and nausea as is described in U.S. Patent No. 4,031,894, e.g., preferably, scopolamine.

Other suitable drugs include the coronary vasodilators described in U.S. Patent No. 3,742,951 such as compounds having nitrate moiety. Some suitable coronary vasodilators as well as other suitable systemic drugs are disclosed in U.S. Patent Nos. 3,996,934 and 4,573,996, and the portions of both of these describing same are incorporated herein by reference thereto.

In the embodiment of the invention shown in FIG. 7, particularly since an active agent is being used, it is necessary to provide a seal in some form to maintain the active agent in a pure and uncontaminated form. This can be accomplished by sealing the entire applicator itself in a separate sealed package, but in the preferred embodiment a seal is provided within the applicator as shown in FIG. 7.

Firstly, in order to seal the carrier layer 32', a small amount of adhesive can be placed between the overlapping portions 34' of the carrier portions 32a' and 32b'. The amount of adhesive should be such as to provide a seal between these two portions of carrier layer 32 while at the same time not interfering with the overall function of this device, i.e., so as to still permit the end of overlying portion of carrier layer 32b' from being peeled back, i.e., the end should remain free of adhesive for that purpose, and the adhesive should be displaced within the overlapping portion 34'.

In order to further seal the applicator, it is thus necessary to provide some form of seal around the periphery of the active agent carrier 40 defined by the film layer 28'. A preferred method of accomplishing this is to provide a "peelable seal" between the outer periphery of the portions of carrier layer 32a' and 32b' and the liner layer 22'. As in shown in FIG. 7 and discussed above, it has already been discussed that the removal of liner layer 22' can be facilitated by extending a tab portion 24' of the liner layer 22' beyond the film layer 28, and having a corresponding overlapping portion of the adjacent portion of carrier layer 32a' corresponding thereto. By extending this around the entire periphery of the applicator itself, it is thus possible to create a peelable seal between these adjacent overlying external peripheral portions of the liner layer 22' and the corresponding portions of carrier layer 32a' and 32b'. The creation of a "peelable seal" around this peripheral edge can be accomplished in the same manner disclosed in U.S. Patent No. 4,710,191, the disclosure of which is incorporated herein by reference thereto.

In particular, in that patent it is disclosed that such a "peelable seal" can be created by the incorporation of a release coating on the inner surface of a layer such as liner layer 22' so as to render the heat-sealed area then created between these two layers, in this case between liner layer 22' and the portions of carrier layer 31a' and 32b', by weakening the thermal bond created therein. It is also disclosed in this patent that one alternative for achieving this result is to employ two different materials as the inner layers for the liner layer 22 and the carrier layer 32, such as polyethylene and polypropylene. In such a case no release coating is then required on the inner surface of the liner layer 22, at least for the purposes of creating such a peelable seal at these locations around the remainder of the periphery thereof.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. An applicator for applying a film layer to the skin or mucosa of a patient comprising a liner layer including a first surface and a second surface, said first surface of said liner layer comprising a releasable surface, a film layer including a first surface and a second surface, said first surface of said film layer being releasably disposed on said releasable surface of said liner layer, and a carrier layer disposed in contact with said second surface of said film layer, characterized by said carrier layer comprising a first portion (32a) and a second portion (32b), said first portion of said carrier layer covering a first portion of said second surface of said film layer (28), and including a first adhesive portion (29a) and a first tab portion (32d) free of adhesive, said second portion of said carrier layer covering a second portion of said second surface of said film layer, and including a second adhesive portion (29b) and a second tab portion (32b) free of adhesive, said first and second tab portions overlapping each other (34), whereby upon removal of said liner layer and application of said first surface of said film layer to said skin or mucosa of said patient said carrier layer can be readily removed from said second surface of said film layer by removal of said first and second portions of said carrier layer by pulling on said first and second tab portions, respectively.

2. The applicator of claim 1, further characterized by an active agent carrier (40') disposed on said first surface of said film layer and being releasably disposed on said releasable surface of said liner layer, whereby upon application of said first layer of said film layer to said skin or mucosa said active agent carrier is applied therewith.

3. The applicator of claim 1 or 2 characterized in that said first adhesive portion (29a) and said second adhesive portion (29b) of said carrier layer includes substantially all of said second surface of said film layer except for said overlapping portion (34) of said first and second tab portions (32d, 32b).

4. The applicator of claim 1, 2 or 3 characterized in that said liner layer includes a liner layer tab portion (24) whereby said liner layer may be readily removed from said film layer.

5. The applicator of claim 1, 2 or 3 characterized in that a portion of said first portion of said carrier layer (32a) extends beyond said film layer so as to be directly adjacent to said liner layer.

6. The applicator of claim 5 characterized in that at least a portion of said first portion of said carrier layer which is adjacent to said liner layer (24) is free of adhesive, thereby facilitating removal of said liner layer from said film layer at that point.

7. The applicator according to any of the previous claims characterized in that said overlapping area of said first and second tab portions of said first and second portions of said carrier layer is substantially located at the center of said applicator.

8. The applicator of claim 7 further characterized by adhesive means between said overlapping portions (34) of said first and second portions of said carrier layer so as to create a seal therebetween.

9. The applicator of claim 8 characterized in that said carrier layer has a dimension which is greater than the dimension of said film layer around the entire periphery of the film layer, and wherein said liner layer also has a dimension which is greater than that of said film layer around the entire periphery of said film layer, and including sealing means around said entire peripheral area of said carrier layer and said liner layer.

10. The applicator of claim 9 characterized in that said carrier layer and said liner layer comprise the same plastic material, said second surface of said liner layer comprises a releasable surface, and wherein said sealing means comprises a heat seal between said carrier layer and said liner layer.

11. The applicator of claim 2 characterized in that said active agent carrier comprises a reservoir (44') containing said active agent, and release means (42') for the controlled release of said active agent from said reservoir.

12. The applicator of claim 11 characterized in that said active agent carrier includes an active agent impermeable barrier layer (48') between said reservoir and said film layer.

13. The applicator of claim 11 characterized in that said release means comprises an active agent permeable membrane layer (42') formed on said reservoir.

F I G. 1

(PRIOR ART)

F I G. 2

(PRIOR ART)

FIG. 3

FIG. 4

FIG. 5

FIG. 6

EP 0 400 949 A2

FIG. 7

F I G. 8

F I G. 9

F I G. 10